# FASCICULE DE BREVET EUROPEEN

(11) **EP 0 554 165 B1**
(45) Date de publication et mention de la délivrance du brevet: **06.12.1995**
(21) Numéro de dépôt: 93400188.4
(22) Date de dépôt: 26.01.1993
(51) Int. Cl.: C07C 17/20, C07C 19/08

(54) **Procédé de fabrication du 1,1,1,2-tétrafluoroéthane**
Verfahren zur Herstellung von 1,1,1,2-Tetrafluorethan
Process for the preparation of 1,1,1,2-tetra-fluoroethane

(30) Priorité: 30.01.1992 FR 9201035
(43) Date de publication de la demande: 04.08.1993
(73) Titulaire: ELF ATOCHEM S.A., 92800 Puteaux (FR)
(72) Inventeur: Galland, Jean-Michel, F-69390 Vernaison (FR); Guiraud, Emmanuel, F-69230 Saint-Genis Laval (FR); Schirmann, Jean-Pierre, F-75011 Paris (FR)
(74) Mandataire: Leboulenger, Jean

(56) Documents cités:
- EP-A- 0 449 617
- WO-A-90/08755
- FR-A- 2 433 500

## Description

La présente invention concerne un procédé continu de fabrication du 1,1,1,2-tétrafluoroéthane à partir de 2-chloro-1,1,1-trifluoroéthane et d'acide fluorhydrique HF.

Le 1,1,1,2-tétrafluoroéthane, connu dans le métier sous la désignation 134a, est principalement destiné à remplacer le dichlorodifluorométhane (CFC 12) dans ses applications en réfrigération.

Sa fabrication par fluoration en phase gazeuse du 2-chloro-1,1,1-trifluoroéthane (connu dans le métier sous la désignation 133a) a déjà fait l'objet de nombreux brevets.

Le brevet US 4 158 675 décrit un procédé de fabrication de 134a par réaction en phase vapeur à température élevée d'un haloéthane de formule CX₃CH₂Cl où X représente Br, Cl ou F, avec de l'HF en présence d'un catalyseur à base d'oxyde de chrome ; le 134a produit, contenant du 2-chloro-1,1-difluoroéthylène comme impureté, est ensuite mis en contact avec de l'HF sur un même catalyseur à base d'oxyde de chrome, à une température comprise entre 100 et 275°C de façon à réduire la teneur en haloéthylène. L'exemple fourni dans ce brevet (durée de l'essai : 3 heures) ne donne pas d'indication sur le recyclage éventuel des réactifs 133a et HF non convertis.

La demande de brevet JP 55 27138/80 décrit un procédé de fabrication de 134a par réaction du 133a avec de l'HF dans une proportion molaire de 1 sur 3 à 20 en présence d'un composé inorganique du chrome III à une température de 300 à 450°C. Les exemples ne donnent pas d'indication sur le recyclage éventuel des réactifs non convertis et la durée des essais.

Le brevet FR 2 433 500 décrit un procédé de fabrication de 134a par réaction du 133a avec HF en présence d'un composé minéral de chrome III, caractérisé en ce que l'on introduit dans le système de réaction de 0,002 à 0,05 mole d'oxygène par mole de 133a. Les exemples ne donnent pas d'indication sur le recyclage éventuel des réactifs non convertis. Les résultats obtenus lors de certains essais conduits en présence d'oxygène montrent des performances de réaction stables sur 85 heures.

La demande de brevet EP 328 127 recommande, comme catalyseur, l'utilisation en présence d'oxygène d'autres métaux que le chrome, à savoir Co, Mn, Ni, Pd, Ag et/ou Ru sur AlF₃, de manière à minimiser l'oxydation en chlore et en eau de l'acide chlorhydrique formé conduisant à une perte de sélectivité et un risque de corrosion. Les exemples fournis dans cette demande de brevet ne donnent pas d'indication sur le recyclage éventuel des réactifs non convertis. Les résultats obtenus lors de certains essais (menés avec oxygène, sur des catalyseurs ne contenant pas de chrome) montrent des performances de réaction stables sur 9 heures (exemple 1) et 19 heures (exemple 2).

La demande de brevet EP 331 991 a pour objet un procédé de fabrication du 134a consistant à mettre en contact en phase gazeuse entre 300 et 500°C du 133a et de l'HF sur un catalyseur contenant au moins un métal ayant un degré d'oxydation supérieur à zéro et sélectionné parmi les métaux des groupes VIII, VII B, III B, I B et/ou les métaux ayant un nombre atomique de 58 à 71, puis à séparer le 134a du flux gazeux sortant. Les exemples illustrant ce procédé ne donnent pas d'indication sur le recyclage éventuel des réactifs non convertis. Une chute d'activité apparaît après 38 heures (exemple 1) ou 21 heures (exemple 2).

Dans la demande de brevet JP 262 946/89 il est indiqué que les méthodes connues de maintien de l'activité d'un catalyseur de fluoration, telles que l'addition continue de chlore ou d'oxygène, ne conviennent pas dans le cas de la fluoration d'un hydrocarbure halogéné contenant de l'hydrogène (tel que le 133a) du fait de la perte de sélectivité observée. Cette publication propose donc un procédé de régénération périodique du catalyseur à l'oxygène consistant en ce que, lorsque l'activité du catalyseur décroît au cours de la réaction, l'introduction des réactifs est arrêtée et un gaz contenant de l'oxygène est alimenté au système réactionnel pour réactiver le catalyseur, puis cette alimentation en gaz contenant de l'oxygène est arrêtée et l'alimentation en réactifs est reprise. Les exemples 2 et 4, et les exemples comparatifs 1 et 2 concernent la fluoration du 133a, en utilisant des catalyseurs contenant du chrome, en présence ou absence d'oxygène. On constate une chute d'activité de ces catalyseurs. Les exemples fournis dans cette publication ne donnent pas d'indication sur le recyclage éventuel des réactifs non convertis.

Dans la demande de brevet JP 172 933/90 ayant pour objet un procédé de fluoration du 133a, il est indiqué que les catalyseurs traditionnels, tels que l'oxyde de chrome seul, ont une faible activité à basse température et une durée de vie courte à haute température. Elle propose donc d'effectuer la réaction entre le 133a et l'HF en présence d'un catalyseur de fluoration à base d'halogénures ou oxydes de chrome et d'au moins un élément sélectionné parmi Al, Mg, Ca, Ba, Sr, Fe, Ni, Co et Mn. Elle recommande aussi, conjointement au catalyseur spécifié, une alimentation d'oxygène ou de chlore sur la base de 0,1 à 10 % molaire par rapport au 133a. Tous les exemples fournis utilisent 2 % molaire d'oxygène et sont conduits sans recyclage des réactifs non convertis.

La demande de brevet WO 90/08755 décrit un procédé amélioré pour la fabrication du 134a à partir de trichloroéthylène, l'amélioration consistant à conduire la réaction de fluoration catalytique dans une zone réactionnelle unique alimentée en trichloroéthylène, en HF et en 133a recyclé, la réaction pouvant être effectuée en présence ou absence d'oxygène. Les exemples fournis sont conduits sans recyclage des réactifs non convertis.

La demande de brevet EP 408 005 décrit un procédé de fabrication de 134a par réaction en phase gazeuse de trichloroéthylène et d'acide fluorhydrique en présence de 133a, le ratio molaire du trichloroéthylène au 133a allant de 5/95 à 50/50, et en présence d'un catalyseur comportant du trioxyde de chrome supporté sur AlF₃. Les exemples fournis sont conduits sans recyclage des réactifs non convertis, à l'exception de l'exemple 6. Cet exemple d'une durée de 6 heures indique une séparation des composants du milieu réactionnel entre, d'une part, des "légers" dont le 134a et, d'autre part, des "lourds" et du 133a qui sont recyclés au réacteur, mais il ne donne ni les conditions opératoires de cette séparation, ni l'effet de ce recyclage sur le catalyseur, ne permettant pas de reproduire l'exemple.

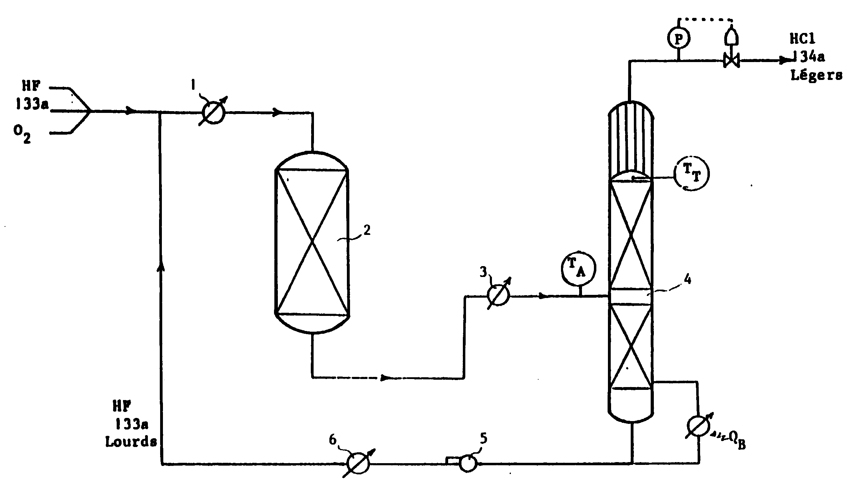

La demande de brevet EP 446 869 décrit un procédé de fabrication de 133a par fluoration en phase gazeuse du trichloroéthylène conduite en présence de diluants inertes tels que les gaz issus de la réaction de fluoration du 133a en 134a. La mise en oeuvre de ce procédé intégré à une fabrication de 134a amène, comme indiqué sur la figure 2 de cette demande de brevet, à conduire la réaction de fluoration du 133a en 134a en présence d'un recyclage de réactifs 133a et HF non convertis. Cependant, les conditions de séparation des produits et réactifs et les effets du recyclage ne sont pas indiqués ; les exemples fournis concernent des essais effectués en l'absence de recyclage, l'alimentation en réactifs étant reconstituée.

Les demandes de brevet EP 449 614 et EP 449 617 décrivent des procédés de fabrication du 134a par fluoration du trichloréthylène en deux étapes réactionnelles (fluoration du trichloréthylène, fluoration du 133a) effectuées en série (fluoration du trichloréthylène, puis du 133a) ou en série inverse (fluoration du 133a, puis du trichloréthylène). Dans les deux cas, ces procédés amènent à conduire la réaction de fluoration du 133a en 134a en présence d'un recyclage de réactifs 133a et HF non convertis. Cependant, les conditions de séparation des produits et réactifs et les effets du recyclage ne sont pas indiqués ; dans les deux demandes de brevet, les exemples fournis concernent des essais effectués en l'absence de recyclage, l'alimentation en réactifs étant reconstituée et la durée des essais n'est pas indiquée.

Dans la fluoration catalytique du 133a, le taux de conversion du 133a en 134a est limité par la thermodynamique. Typiquement, pour un ratio molaire HF/133a égal à 2 à l'entrée du réacteur et une température réactionnelle de 400°C, l'équilibre thermodynamique correspond à des taux de conversion du 133a de 20 % et de l'HF de 10 %. Le flux sortant du réacteur contient donc majoritairement des réactifs non transformés (133a et HF) qu'il est primordial de recycler. Pour cela, on peut conformément aux techniques classiques séparer puis purifier les principaux constituants du flux sortant du réacteur, notamment le 133a et l'HF non convertis pour en éliminer avant recyclage en réaction les impuretés gênantes (telles que des sous-produits organiques ou de l'eau) qui sont générées en réaction ou apportées par les matières premières et qui sont susceptibles d'entraîner une désactivation du catalyseur ou de générer des corrosions.

Il a maintenant été trouvé que l'application de cette technique classique n'est pas nécessaire dans le cas de la fabrication du 134a et que, sous certaines conditions, on peut recycler directement au réacteur un mélange brut contenant l'essentiel du 133a et de l'HF non transformés, après séparation des produits HCl et 134a.

Ce résultat est particulièrement inattendu à la revue de l'art existant où, en l'absence de tout recyclage, c'est-à-dire avec des réactifs de puretés contrôlée, les performances de réaction obtenues (conversion, sélectivité, durée de vie) ne sont pas économiques.

L'invention a donc pour objet un procédé continu de fabrication du 134a à partir de 133a et d'HF en phase gazeuse en présence d'un catalyseur à base de chrome, caractérisé en ce que le flux gazeux sortant du réacteur est soumis à une distillation pour séparer en tête un flux contenant la quasi-totalité de l'acide chlorhydrique et au moins 90 % du 134a produits par la réaction et en pied un flux contenant au moins 90 % des réactifs non transformés (133a et HF) présents dans le flux gazeux sortant du réacteur, et que l'on recycle directement au réacteur le flux récupéré en pied de distillation, sans aucune opération de purification.

Comme on pouvait s'y attendre, le recyclage des réactifs non convertis directement au réacteur, en l'absence de purification particulière du recyclat, conduit à un certain taux d'accumulation d'eau et de sous-produits organiques dans ce recyclat. Curieusement, en régime établi, la teneur en eau et en ces sous-produits organiques se stabilise dans un état stationnaire et n'obère pas les performances du catalyseur :
- taux de conversion du 133a proche de l'état d'équilibre thermodynamique de la réaction 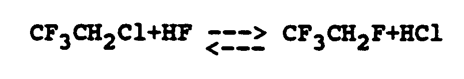
- sélectivité en 134a élevée, typiquement supérieure à 98 %.

Par ailleurs, on observe que la teneur en eau de la boucle de recyclage se stabilise curieusement à une valeur faible.

Dans la mise en oeuvre du procédé selon l'invention, ces performances (activité, sélectivité) restent stables pendant au moins plusieurs centaines d'heures ; ceci permet d'éviter des opérations fréquentes de remplacement ou de régénération du catalyseur, génératrices de coûts élevés en investissement et frais opératoires. Le procédé selon l'invention est d'autant plus économique qu'il n'implique pas d'opérations de purification du flux à recycler.

Le catalyseur à base de chrome, utilisé conformément à la présente invention, peut être un catalyseur massique ou un catalyseur supporté.

Comme catalyseurs massiques, on utilise essentiellement comme matériaux de départ des oxydes de chrome, de préférence amorphes, mais il peut s'agir aussi de sels de chrome tels que le sulfate de chrome sous différentes formes commerciales. La mise en forme de ces catalyseurs peut faire appel à des techniques très diverses bien connues de l'homme de l'art. La forme du catalyseur n'est pas critique et l'on peut utiliser aussi bien des pastilles, des granulés, des poudres ou encore des microsphères. La quantité de chrome, exprimée en métal avant traitement à l'acide fluorhydrique, est élevée, généralement comprise entre 20 % et 68 % en poids. Le reste de la composition du catalyseur est généralement de l'oxygène, nais peut contenir aussi d'autres éléments (par exemple le silicium, le carbone, les métaux de transition, les alcalino-terreux, les terres rares ou de l'uranium) et d'une façon plus générale des métaux appartenant aux groupes VIII, VII B, VI B, III B, II B et I B de la Classification Périodique des éléments.

Pour les catalyseurs supportés, on peut utiliser comme supports des matériaux tels que des charbons actifs, de l'alumine, du trifluorure d'aluminium, ou encore de l'alumine partiellement fluorée. Par alumine partiellement fluorée, on entend une composition riche en fluor et contenant de l'aluminium, de l'oxygène et du fluor dans des proportions telles que la quantité du fluor exprimée en AlF₃ représente au moins 50 % du total. Le support peut se présenter sous forme de poudre ou de pastilles.

Les catalyseurs supportés peuvent être préparés selon les techniques bien connues de l'homme de l'art. Par exemple, on peut imprégner du charbon actif ou de l'alumine partiellement fluorée avec une solution contenant au moins un sel de chrome ou de l'acide chromique CrO₃ et éventuellement un sel d'un autre métal, par exemple, alcalino-terreux, métaux de transition, uranium, terres rares et d'une façon plus générale les métaux des groupes VIII, VII B, VI B, III B, II B et I B. La teneur en chrome de ces catalyseurs supportés est en général inférieure à 20 % en poids et on utilise en général des teneurs comprises entre 4 et 10 %.

Le catalyseur peut aussi être préparé par coprécipitation des constituants finaux sous leur forme d'hydroxydes métalliques, puis séché, et calciné pour former des oxydes mixtes selon les techniques bien connues de l'homme de l'art.

Les catalyseurs au chrome utilisés, massiques ou supportés, sont prétraités avant mise en réaction, par de l'HF seul ou, plus généralement, mélangé à un gaz inerte tel que l'azote. Ce traitement est en général réalisé pendant une durée de 1 à 24 heures et à une température comprise entre 200 et 450°C.

La réaction proprement dite du 133a avec HF en présence d'un catalyseur à base de chrome selon l'invention peut être conduite dans un domaine de température compris entre 300 et 450°C, préférablement entre 330 et 400°C, avec un temps de contact compris entre 0,1 seconde et 60 secondes, préférablement de 5 à 30 secondes.

La pression sous laquelle la réaction peut être conduite est comprise entre la pression atmosphérique et 30 bars absolus. On opère préférablement sous une pression allant de 10 à 15 bars absolus qui permet de réaliser économiquement la séparation de l'HCl anhydre du 134a. L'utilisation d'une pression élevée permet par ailleurs d'améliorer considérablement la productivité.

La quantité d'acide fluorhydrique utilisé est au moins égale à la stoechiométrie, mais le rapport HF/133a à l'alimentation de la réaction est avantageusement compris entre 1 et 10, de préférence 2 à 5.

Pour certains catalyseurs, il peut être intéressant de travailler en présence d'oxygène afin d'améliorer la durée de vie du catalyseur. La quantité d'oxygène utilisée, rapportée au 133a alimentant la réaction, peut varier entre 0,1 et 5 % molaires. L'oxygène peut être introduit dans la zone réactionnelle, soit seul, soit en mélange avec un inerte tel que l'azote et bien entendu sous forme d'air. L'utilisation d'oxygène n'est pas sans inconvénients pour la sélectivité de la réaction. On observe en effet l'apparition dans le produit de réaction de CO, CO₂, et via sans doute la réaction de Deacon entre l'HCl formé et l'oxygène (donnant du chlore et de l'eau) la formation de sous-produits tels que le 1,1-dichloro-2,2,2-trifluoroéthane (HCFC-123), le 1-chloro-1,2,2,2-tétrafluoroéthane (HCFC-124) et le pentafluoroéthane (HCFC-125). Il a cependant été constaté que l'emploi d'oxygène ou d'air ne perturbe en rien la séparation en aval de la réaction et que les réactifs non convertis (HF, 133a) et les impuretés lourdes qui les accompagnent peuvent être recyclés en réaction après séparation dans les conditions spécifiées sans que l'activité du catalyseur baisse. On atteint un régime stationnaire permettant de fonctionner en boucle durant au moins plusieurs centaines d'heures.

La réaction du 133a avec HF peut être réalisée dans différents types de réacteurs suivant le catalyseur utilisé, ses propriétés mécaniques et sa résistance à l'attrition. On peut opérer soit en lit fixe, soit en lit fluide et soit dans un ou plusieurs réacteurs. Les matériaux utilisés doivent résister à la corrosion du milieu et doivent être, par exemple, de l'Inconel ou de l'Hastelloy.

Le flux gazeux sortant du réacteur de fluoration comprend principalement de l'HF, du 133a, du 134a et de l'HCl. Conformément à la présente invention, ce flux gazeux est soumis à une séparation par distillation de façon à récupérer, d'une part, la quasi-totalité de l'HCl et au moins 90 % du 134a présents dans ce flux et, d'autre part, au moins 90 % du 133a et de l'HF qui sont recyclés directement en réaction.

Cette séparation peut être effectuée par distillation en une ou deux étapes, c'est-à-dire en séparant l'HCl puis le 134a ou directement, et plus simplement, en une étape en séparant l'HCl et le 134a en mélange. Dans ce cas on obtient en tête de distillation l'essentiel de l'HCl et du 134a et en pied l'essentiel du 133a et de l'HF.

Cette distillation est préférablement réalisée dans une colonne en acier inoxydable qui peut être équipée de plateaux ou d'un garnissage. La distillation peut être menée sous une pression pouvant aller de 1 à 30 bars absolus, suivant la pression sous laquelle est effectuée la réaction de fluoration catalytique. La température d'alimentation du mélange réactionnel peut aller de 20 à 150°C. La température de tête dépend bien sûr de l'efficacité de séparation recherchée et varie en fonction de la pression ; elle est d'environ 5°C sous 3 bars absolus et d'environ 55°C sous 15 bars absolus.

A pression fixée, la température de tête sert à régler la teneur en 133a et HF du flux de tête tandis que le taux de rebouillage en pied sert à régler l'élimination de l'HCl et du 134a. L'HF, passant en tête est essentiellement relié aux azéotropes existants avec le 134a et le 133a.

On observe que si l'on élimine pas l'essentiel de l'HCl et 90 % au moins du 134a produits en réaction, la productivité, mais également la sélectivité, décroissent en réaction. De même, le fait de ne pas séparer et recycler directement au moins 90 % du 133a et de l'HF présents en sortie de réaction contraint inutilement à retraiter ces produits en aval de la boucle réactionnelle.

On opère de préférence la réaction et cette distillation sous une pression comprise entre environ 10 et 15 bars absolus. En effet, dans ces conditions, le mélange HCl/134a est lui aussi séparable économiquement par distillation avec production d'acide chlorhydrique anhydre. Par contre, à une pression de l'ordre de 2 à 3 bars absolus, le mélange HCl/134a issu de cette distillation doit généralement être traité à l'eau pour éliminer l'HCl.

Le flux des réactifs non convertis, obtenu en pied de la colonne de distillation, n'est soumis à aucun traitement particulier de purification ou d'élimination d'impuretés organiques ou minérales. Ce flux, majoritairement composé de 133a et d'HF, contient donc diverses impuretés organiques, des traces d'eau (de l'ordre de 1000 ppm en poids ou moins) et éventuellement une faible proportion des produits de réaction (134a et HCl) non séparés. Ce flux est directement recyclé au réacteur de fluoration. Les réactifs frais (133a et HF) sont par ailleurs alimentés en un point quelconque de l'ensemble réaction-séparation-recyclage, dans des proportions permettant de compenser la production nette de 134a et d'HCl.

Les réactifs frais peuvent être introduits soit avant distillation en aval de la réaction pour refroidir les gaz, soit dans le flux des réactifs non convertis recyclés en réaction.

Le 133a et l'HF éventuellement présents en faible proportion dans le flux de 134a et d'HCl issu de tête de distillation peuvent être séparés ultérieurement des produits de réaction par les méthodes connues en soi et recyclés au réacteur.

Les exemples suivants illustrent l'invention sans la limiter. Ils ont été réalisés sur une installation représentée sur la figure unique annexée. Cette installation comprend un réacteur (2) en Inconel d'un volume utile de 100 litres et une colonne de distillation (4) en inox 316 L ayant un diamètre intérieur de 150 mm, une hauteur de 7800 mm et munie d'un garnissage Multiknit en Inox 316 L.

Les réactifs frais et le flux des réactifs non convertis (recyclage) sont alimentés au réacteur après préchauffage dans un préchauffeur électrique (1). L'effluent gazeux sortant du réacteur est refroidi dans un échangeur réfrigérant (3), puis introduit dans la colonne de distillation.

En tête de la colonne de distillation, on récupère l'HCl, le 134a et les produits légers, et en pied le 133a, l'HF et les produits plus lourds. Ce flux de réactifs non convertis est recyclé au réacteur par l'intermédiaire d'une pompe (5) et d'un évaporateur (6) .

A l'alimentation du préchauffeur (1), on effectue l'appoint en HF et 133a frais, et éventuellement une alimentation en oxygène.

L'HF utilisé, de qualité technique, a une pureté de 99,9 % poids contenant comme impureté principale de l'eau à hauteur d'environ 500 à 1000 ppm.

Le 133a utilisé a une pureté de 99,95 poids

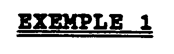

Le catalyseur utilisé est un catalyseur Ni + Cr/AlF₃ préparé par imprégnation d'une alumine partiellement fluorée (teneur pondérale en AlF₃ supérieure à 78 %) avec de l'acide chromique et du chlorure de nickel hexahydraté, puis réduction au méthanol. Ses principales caractéristiques physico-chimiques sont les suivantes :
- composition chimique (pondérale)

| | |
|---|---|
| . fluor | 46,6 % |
| . aluminium | 31,8 % |
| . nickel | 3,6 % |
| . chrome | 3,4 % |
| . oxygène | 10,2 % |
| . chlore | 4,4 % |

- propriétés physiques :

| | |
|---|---|
| . surface BET | 40 m²/g |
| . granulométrie | billes de 1 à 2 mm |

Ce catalyseur a été préalablement traité à 350°C pendant 10 heures au moyen d'un mélange d'acide fluorhydrique et d'azote à 5 % molaire d'HF, puis à 400°C pendant 5 heures au moyen d'un mélange d'acide fluorhydrique et d'azote à 10 % molaire d'HF.

La fluoration du 133a avec ce catalyseur a été effectuée dans les conditions opératoires suivantes :
**a)** Réaction

| | |
|---|---|
| - température de réaction | 350°C |
| - pression | 3,1 bars absolus |
| - temps de contact | 9 secondes |
| - rapport molaire HF/133a à l'alimentation du réacteur | 4 |
| - rapport molaire O₂/133a à l'alimentation du réacteur | 0,15 % |

**b)** Séparation
La colonne à distiller destinée à traiter le flux en sortie du réacteur a été réglée de'la façon suivante :

| | |
|---|---|
| - température d'alimentation, T_{A} | 50°C |
| - température en tête, T_{T} | 5°C |
| - chaleur fournie au bouilleur Q_{B} | 2,8 kW |
| - pression | 3,1 bars absolus |

Ces conditions de fonctionnement ont permis d'obtenir en tête un flux de 134a et HCl et en pied un flux de 133a et HF tels que :
- les teneurs en 134a et HCl du flux de 133a et HF recyclés étaient inférieures à 100 ppm, ce qui correspond à des taux de récupération de plus de 99,7 % pour ces deux produits
- les teneurs molaires en 133a et HF dans le flux de 134a et HCl étaient respectivement de 2,3 % et 14,4 %, ce qui correspond à des taux de récupération de 98,7 % pour le 133a et 98,3 % pour l'HF.

Après 700 heures de fonctionnement, les performances observées sont les suivantes :
**a)** Conversion par passe du 133a = 19,5 %. On entend par conversion le rapport entre le 133a consommé et le 133a entrant au réacteur.
**b)** Sélectivité par passe en 134a = 98,8 %. On entend par sélectivité en 134a le rapport entre le 134a produit et le 133a consommé.

On observe dans le flux des produits de réaction récupéré en tête de la colonne de distillation les impuretés principales suivantes : CF₃-CHClF (124), CHCl=-CF₂ (1122), CF₃-CHF₂ (125), CF₃-CH₃ (143a), CClF₃ (13), CHF₃ (23), O₂, CO et CO₂.
**c)** Productivité en 134a = 97 g/h/litre de catalyseur
**d)** Teneur en eau dans le flux recyclé : plusieurs analyses du flux recyclé ont été effectuées tout au long de cet essai afin de déterminer sa teneur en eau (méthode Karl Fischer). Les résultats ont montré qu'il n'y avait pas d'accumulation d'eau et que la teneur en H₂O dans le flux recyclé était comprise entre 600 et 800 ppm en poids.
**e)** Teneur en impuretés organiques dans le recyclat : plusieurs analyses du flux recyclé ont été effectuées tout au long de cet essai afin de déterminer la teneur en impuretés organiques dans ce flux. Les résultats ont montré qu'il n'y avait pas d'accumulation et que la teneur en impuretés était stable. Cette teneur, exprimée en ppm molaires par rapport au flux total recyclé, est inférieure à 300 ppm. Les impuretés principales sont : CHCl₂-CF₃ (123) et CHClF-CF₃ (124).

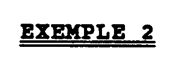

Le catalyseur utilisé dans cet exemple est un catalyseur au chrome massique dont les principales caractéristiques physico-chimiques sont les suivantes :
- Composition chimique (pondérale)

| | |
|---|---|
| . chrome | 68 % |
| . oxygène | 32 % |

- Propriétés physiques :

| | |
|---|---|
| . granulométrie (diamètre moyen) | 0,5 mm |
| . surface BET | 50 m²/g |

Ce catalyseur, préalablement traité au moyen d'un mélange d'HF et N₂ (30 % molaire d'HF) à 350°C pendant 5 heures, a été mis en oeuvre en l'absence d'oxygène dans les conditions opératoires suivantes :
**a) réaction** :

| | |
|---|---|
| - température de réaction | 350°C |
| - pression | 3 bars absolus |
| - temps de contact | 7 secondes |
| - rapport molaire HF/133a à l'alimentation du réacteur | 4 |

**b) séparation** :
La colonne de distillation a été réglée de la façon suivante :

| | |
|---|---|
| - température d'alimentation, T_{A} | 50°C |
| - température en tête, T_{T} | 3°C |
| - chaleur fournie au bouilleur, Q_{B} | 4,7 kW |
| - pression | 3 bars absolus |

Dans ces conditions, les teneurs en 134a et HCl du flux de 133a et HF recyclés étaient inférieures à 100 ppm ce qui correspond à des taux de récupération en 134a et HCl de plus de 99,5 %.

D'autre part, les teneurs molaires en 133a et HF dans le flux de 134a et HCl récupéré en tête de la colonne de distillation étaient respectivement de 1,4 % et 10,7 %, ce qui correspond à des taux de récupération de 99,2 % pour le 133a et 98,7 % pour l'HF.

Après 260 heures de fonctionnement, les performances observées sont les suivantes :

| | |
|---|---|
| **a)** Conversion par passe du 133a | 15 % |
| **b)** Sélectivité par passe en 134a | 99,5 % |

On observe dans le flux des produits de réaction récupéré en tête de la colonne de distillation les impuretés principales suivantes : CF₃-CHClF (124), CHCl=CF₂ (1122), CF₃-CHF₂ (125), CF₃-CH₃ (143a), CClF₃ (13), CHF₃ (23).
**c)** Productivité en 134a = 92 g/h/litre de catalyseur
**d)** Teneur en eau dans le flux recyclé : plusieurs analyses du flux recyclé ont été effectuées tout au long de cet essai afin de déterminer sa teneur en eau. Les résultats ont montré qu'il n'y avait pas d'accumulation d'eau et que la teneur en H₂O dans le flux recyclé était comprise entre 500 et 700 ppm en poids.
**e)** Teneur en impuretés organiques dans le recyclat : plusieurs analyses du flux recyclé ont été effectuées tout au long de cet essai afin de déterminer la teneur en impuretés organiques dans ce flux. Les résultats ont montré qu'il n'y avait pas d'accumulation et que la teneur en impuretés était stable. Cette teneur, exprimée en ppm molaires par rapport au flux total recyclé, est inférieure à 400 ppm. La principale impureté est le CHCl₂CF₃ (123).

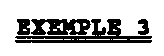

Le catalyseur utilisé est un catalyseur Ni+Cr/AlF₃, préparé de la même façon qu'à l'exemple 1, mais avec des teneurs en chrome et en nickel plus élevées. Ses principales caractéristiques physico-chimiques sont les suivantes :
- Composition chimique (pondérale)

| | |
|---|---|
| . fluor | 41,5 % |
| . aluminium | 28,3 % |
| . nickel | 6,4 % |
| . chrome | 5,9% |
| . oxygène | 10,3 % |
| . chlore | 7,6 % |

- Propriétés physiques :

| | |
|---|---|
| . surface BET | 50 m²/g |
| . granulométrie | billes de 1 à 2 mm |

Ce catalyseur a été préalablement traité au moyen d'un mélange d'acide fluorhydrique et d'azote à 5 % molaire d'HF entre 260°C et 300°C pendant 10 heures, puis à 10 % molaire d'HF à 350°C pendant 4 heures.

La fluoration du 133a est effectuée dans les conditions opératoires suivantes :
**a) réaction :**

| | |
|---|---|
| - température de réaction | 350°C |
| - pression | 12 bars absolus |
| - temps de contact | 24 secondes |
| - rapport molaire HF/133a à l'alimentation du réacteur | 2 |
| - rapport molaire O₂/133a à l'alimentation du réacteur | 0,7 % |

**b) séparation :**
La colonne de distillation a été réglée de la façon suivante :

| | |
|---|---|
| - température d'alimentation, T_{A} | 100°C |
| - température en tête, T_{T} | 63°C |
| - chaleur fournie au bouilleur, Q_{B} | 12,5 kW |
| - pression | 12 bars absolus |

Ces conditions de fonctionnement ont permis d'obtenir en tête un flux de 134a et HCl et en pied un flux de 133a et d'HF tels que :
- dans le flux recyclé les teneurs molaires en HCl et 134a étaient respectivement de 0,1 % et 0,5 %, ce qui correspond à des taux de récupération d'environ 98,2 % pour l'HCl et 91 % pour le 134a ;
- les teneurs molaires en 133a et HF dans le flux récupéré en tête de colonne de distillation étaient respectivement de 3,0 % et 13 %, ce qui correspond à des taux de récupération de 98,8 % pour le 133a et 97,7 % pour l'HF.

Après 600 heures de fonctionnement, les performances observées sont les suivantes :

| | |
|---|---|
| **a)** Conversion par passe du 133a | 15 % |
| **b)** Sélectivité par passe en 134a | 98,8 % |

Les impuretés observées dans le flux des produits de réaction récupéras en tête de la colonne de distillation sont identiques à celles citées dans l'exemple 1.
**c)** Productivité en 134a = 180 g/h/litre de catalyseur
**d)** Teneur en eau dans le flux recyclé : les analyses effectuées tout au long de cet essai ont montré qu'il n'y avait pas d'accumulation d'eau dans le flux recyclé. La teneur en eau dans ce flux est comprise entre 600 et 800 ppm en poids.
**e)** Teneur en impuretés organiques dans le recyclat : les analyses effectuées tout au long de cet essai ont montré qu'il n'y avait pas d'accumulation d'impuretés organiques dans le flux recyclé en réaction. La teneur en impuretés organiques est stable et inférieure à 500 ppm molaires. Les principales impuretés sont identiques à celles citées dans l'exemple 1.

## Revendications

1. Procédé continu de fabrication du 1,1,1,2-tétrafluoroéthane (134a) à partir de, 2-chloro-1,1,1-trifluoroéthane (133a) et d'acide fluorhydrique en phase gazeuse en présence d'un catalyseur à base de chrome, caractérisé en ce que l'on soumet le flux gazeux sortant du réacteur à une distillation pour séparer en tête un flux contenant la quasi-totalité de l'acide chlorhydrique et au moins 90 % du 134a produits par la réaction et en pied un flux contenant au moins 90 % des réactifs non transformés (133a et HF) présents dans le flux gazeux sortant du réacteur, et on recycle directement au réacteur le flux récupéré en pied de distillation, sans aucune opération de purification.

2. Procédé selon la revendication 1, dans lequel la réaction et la distillation sont menées sous une pression allant de 1 à 30 bars absolus, de préférence sous une pression comprise entre environ 10 et 15 bars absolus.

3. Procédé selon la revendication 1 ou 2, dans lequel le catalyseur à base de chrome est un catalyseur massique ou un catalyseur supporté.

4. Procédé selon l'une des revendications 1 à 3, dans lequel la réaction de fluoration est effectuée à une température comprise entre 300 et 450°C, de préférence entre 330 et 400°C.

5. Procédé selon l'une des revendications 1 à 4, dans lequel le temps de contact est compris entre 0,1 et 60 secondes, de préférence entre 5 et 30 secondes.

6. Procédé selon l'une des revendications 1 à 5, dans lequel le rapport molaire HF/133a en entrée de réaction est compris entre 1 et 10, de préférence entre 2 et 5.

7. Procédé selon l'une des revendications 1 à 6, dans le quel on effectue la réaction en présence d'oxygène à raison de 0,1 à 5 moles d'oxygène pour 100 moles de 133a en entrée de réaction.

## Claims

1. Continuous process for the preparation of 1,1,1,2-tetrafluoroethane (134a) from 2-chloro-1,1,1-trifluoroethane (133a) and hydrofluoric acid in the gaseous phase in the presence of a chromium-based catalyst, characterised in that the flow of gas leaving the reactor is subjected to a distillation in order to separate at the top a flow containing almost all the hydrochloric acid and at least 90% of the 134a produced by the reaction and at the bottom a flow containing at least 90% of the unconverted reactants (133a and HF) present in the flow of gas leaving the reactor, and the flow recovered at the bottom of the distillation is recycled directly to the reactor, without any purifying operation.

2. Process according to Claim 1, in which the reaction and the distillation are conducted at a pressure ranging from 1 to 30 bar absolute, preferably at a pressure of between approximately 10 and 15 bar absolute.

3. Process according to Claim 1 or 2, in which the chromium-based catalyst is a bulk catalyst or a supported catalyst.

4. Process according to one of Claims 1 to 3, in which the fluorination reaction is carried out at a temperature of between 300 and 450°C, preferably between 330 and 400°C.

5. Process according to one of Claims 1 to 4, in which the contact time is between 0.1 and 60 seconds, preferably between 5 and 30 seconds.

6. Process according to one of Claims 1 to 5, in which the HF/133a mole ratio entering the reaction is between 1 and 10, preferably between 2 and 5.

7. Process according to one of Claims 1 to 6, in which the reaction is carried out in the presence of oxygen in the ratio of 0.1 to 5 mol oxygen to 100 mol of 133a entering the reaction.

## Patentansprüche

1. Kontinuierliches Verfahren zur Herstellung von 1,1,1,2-Tetrafluorethan (134a), ausgehend von 2-Chlor-1,1,1-trifluorethan (133a) und Fluorwasserstoff in der Gasphase in Gegenwart eines Katalysators auf Basis von Chrom, dadurch gekennzeichnet, daß man den aus dem Reaktor austretenden Gasstrom einer Destillation unterwirft, um am Kolonnenkopf einen Strom, der fast den gesamten Chlorwasserstoff und mindestens 90 % des Produktes 134a aus der Reaktion enthält, und am Kolonnensumpf einen Strom, der mindestens 90 % der nicht umgesetzten Reagenzien (133a und HF) enthält, die in dem aus dem Reaktor austretenden Gasstrom enthalten sind, zu trennen und daß man den am Kolonnensumpf wiedergewonnenen Strom dem Reaktor ohne irgendeinen Reinigungsschritt direkt wieder zuführt.

2. Verfahren nach Anspruch 1, in dem die Reaktion und die Destillation unter einem Druck von 1 bis 30 bar, vorzugsweise unter einem Druck von etwa 10 bis 15 bar, durchgeführt werden.

3. Verfahren nach Anspruch 1 oder 2, in dem der Katalysator auf Basis von Chrom ein Katalysator in Masse oder ein auf ein Trägermaterial aufgebrachter Katalysator ist.

4. Verfahren nach einem der Ansprüche 1 bis 3, in dem die Fluorierungsreaktion bei einer Temperatur zwischen 300 und 450 °C, vorzugsweise zwischen 330 und 400 °C, durchgeführt wird.

5. Verfahren nach einem der Ansprüche 1 bis 4, in dem die Kontaktzeit zwischen 0,1 und 60 Sekunden, vorzugsweise zwischen 5 und 30 Sekunden, liegt.

6. Verfahren nach einem der Ansprüche 1 bis 5, in dem das HF/133a-Molverhältnis zu Beginn der Reaktion zwischen 1 und 10, vorzugsweise zwischen 2 und 5, liegt.

7. Verfahren nach einem der Ansprüche 1 bis 6, in dem man die Reaktion in Anwesenheit von Sauerstoff in einer Menge von 0,1 bis 5 mol Sauerstoff auf 100 mol des Stoffes 133a zu Beginn der Reaktion durchführt.
